# EUROPÄISCHE PATENTSCHRIFT

(11) **EP 2 953 480 B1**
(45) Veröffentlichungstag und Bekanntmachung des Hinweises auf die Patenterteilung: **03.06.2020**
(21) Anmeldenummer: 14702825.2
(22) Anmeldetag: 03.02.2014
(51) Int. Cl.: A23K 10/12, C12N 1/06

(54) **VERBESSERUNG DER BIOVERFÜGBARKEIT VON WERTSTOFFEN AUS MIKROORGANISMEN**
IMPROVING BIOAVAILABILITY OF VALUABLE MATERIALS FROM MICROORGANISMS
AMÉLIORISATION DE LA BIODISPONIBILITÉ DE SUBSTANCES DE VALEUR À PARTIR DE MICROORGANISMES

(30) Priorität: 05.02.2013 EP 13153977; 07.02.2013 DE 102013201978
(43) Veröffentlichungstag der Anmeldung: 16.12.2015
(73) Patentinhaber: Evonik Operations GmbH, 45128 Essen (DE)
(72) Erfinder: RUDINGER, Nicolas, 40215 Düsseldorf (DE); RABE, Christian, 63762 Großostheim (DE)
(74) Vertreter: Evonik Patent Association
(86) Internationale Anmeldenummer: PCT/EP2014/052034
(87) Internationale Veröffentlichungsnummer: WO 2014/122092

(56) Entgegenhaltungen:
- EP-A1- 2 384 647
- WO-A1-01/53512
- WO-A2-2006/124598
- US-A1- 2003 138 477
- US-A1- 2011 045 528
- US-A1- 2012 183 668

## Beschreibung

Die vorliegende Erfindung betrifft ein Verfahren zum schonenden Aufschluss von Wertstoffe enthaltenden Zellen, Nahrungsmittel und Futtermittel, die solche durch schonende Aufschluss erhaltene Wertstoffe enthalten, sowie Tiere, die durch Fütterung mit solchen Futtermitteln erhalten wurden.

Die Bedeutung von mikrobiellen Zellen zur Herstellung von Wertstoffen ist dem Fachmann bekannt. Ein Beispiel für solche Wertstoffe stellen Nahrungsmittelkomponenten dar, insbesondere Lipide wie etwa polyungesättigte Fettsäuren. Für die Herstellung von solchen Wertstoffen spielen neben Bakterien und Hefen insbesondere auch weitere Pilze sowie Algen eine besondere Rolle.

Bestimmte Wertstoffe, insbesondere polyungesättigte Fettsäuren (PUFAs), stellen eine wichtige Komponente für die Ernährung von Mensch und Tier dar. Als Quelle für omega-3-Fettsäuren wurde anfangs vor allem Fisch verwendet. Später wurde entdeckt, dass bestimmte Mikroben heterotroph omega-3-Fettsäuren in großen Mengen produzieren, wobei die Fettsäureproduktion durch Wahl spezifischer Reaktionsparameter vorteilhaft beeinflusst werden kann. Die omega-3-Fettsäuren können anschließend aus den Zellen gewonnen werden oder aber die Zellen in Form von Biomasse direkt in Futter- oder Nahrungsmitteln eingesetzt werden.

Ein Problem bei der Verwendung und Verarbeitung von zahlreichen Wertstoffen, insbesondere von polyungesättigten Fettsäuren, stellt ihre Instabilität gegenüber oxidativen Abbau dar: Sobald der Wertstoff aus den Zellen isoliert wird, ist die Gefahr des oxidativen Abbaus stark erhöht, da der Schutz durch die umgebende Zellmembran nicht mehr gegeben ist. Wenn der Wertstoff jedoch nicht aus den Zellen isoliert wird, ist die Bioverfügbarkeit reduziert, d.h. der Wertstoff kann von dem konsumierenden Tier nicht verwertet werden, da die Zellwand nicht oder nicht in ausreichender Menge gespalten werden kann. Ein Kompromiss besteht entsprechend darin, den Wertstoff erst unmittelbar vor seinem Einsatz bzw. erst bei Herstellung des Futter- oder Nahrungsmittels aus den Zellen freizusetzen. Auf diese Weise wird sichergestellt, dass der Wertstoff auf bestmögliche Weise stabilisiert wird, bevor er seiner Anwendung zugeführt wird. In diesem Sinne wird in US 2012/0183668 A1 beschrieben, dass zur Herstellung von Futtermitteln PUFAs enthaltende Zellen mit anderen Futtermittelinhaltsstoffen vermischt werden und das so erhaltene Gemisch zwecks Herstellung eines Futtermittels anschließend einem Extrusionsverfahren unterworfen wird.

Es hat sich jedoch herausgestellt, dass bei Einsatz der PUFAs enthaltenden Zellen der Zellaufschluss in der Regel nur unzureichend erfolgt bzw. dass zur Erzielung eines zufriedenstellenden Zellaufschlusses sehr harsche Zellaufschlussbedingungen in Form eines hohen mechanischen Energieeintrags gewählt werden müssen, wobei selbst dann nicht sichergestellt ist, dass der Zellaufschluss tatsächlich zufriedenstellend verläuft. Bei dem anzuwendenden hohen Energieeintrag kann es im Übrigen zur Schädigung der PUFAs oder anderer Bestandteile der Futtermittelmischung kommen.

Die Aufgabe der vorliegenden Aufgabe bestand daher darin, ein schonenderes Verfahren zur Herstellung von Futtermitteln, die Wertstoffe, vorzugsweise Lipide, insbesondere polyungesättigte Fettsäuren, enthalten, zur Verfügung zu stellen, wobei eine Schädigung der Wertstoffe bestmöglich vermieden werden sollte.

Überraschenderweise wurde erfindungsgemäß gefunden, dass der mechanische Energieeintrag deutlich reduziert werden kann, wenn vor Durchführung des Zellaufschlussverfahrens die Zellen durch Zuführung von Wasser oder einer wässrigen Lösung aufgequollen werden, so dass die Gefahr der Schädigung des enthaltenen Wertstoffs stark minimiert werden kann. Besonders überraschend konnte hierbei selbst mit relativ geringer Wasserzugabe noch ein ausreichender Aufschluss der Zellen erreicht werden, was insbesondere mit Hinblick auf die anschließende Weiterverarbeitung von besonderem Vorteil ist.

Die Dokumente WO 01/53512, US 2011/045528, WO 2006/124598, US 2003/138477 und US 2012/183668 beschreiben bereits Verfahren, bei welchen PUFAs enthaltende Zellen zwecks Isolation der enthaltenen PUFAs aufgeschlossen werden. Das erfindungsgemäße schonende Zellaufschlussverfahren, bei welchem die Zellen zunächst aufgequollen und anschließend bei niedrigem Energieeintrag aufgeschlossen werden, wird durch diesen Stand der Technik jedoch nicht vorweg genommen.

Ein erster Gegenstand der vorliegenden Erfindung ist daher ein Verfahren zum Aufschluss von Zellen, die Wertstoffe, insbesondere Lipide, vorzugsweise ungesättigte Fettsäuren enthalten, dadurch gekennzeichnet, dass die Zellen vor dem Zellaufschluss aufgequollen werden, wobei bei Durchführung des Zellaufschlusses ein Energieeintrag auf die Zellsuspension von 0,1 - 50 kWh/t erfolgt, dadurch gekennzeichnet, dass das Aufquellen der Zellen dadurch erfolgt, dass ausgehend von einer Zellmasse mit einem Feuchtigkeitsgehalt von weniger als 15 Gew.-% eine Zellsuspension mit einem Feuchtigkeitsgehalt von 30 bis 60 Gew.-% eingestellt wird. Unter "Aufquellen der Zellen" ist erfindungsgemäß zu verstehen, dass Wasser (oder eine wässrige Lösung) in die Zellen eindringt und hierdurch der osmotische Druck in den Zellen erhöht wird.Das Aufquellen der Zellen erfolgt erfindungsgemäß dadurch, dass ausgehend von einer Zellmasse mit einem Feuchtigkeitsgehalt von weniger als 15 Gew.-%, insbesondere 1 - 14 Gew.-%, vorzugsweise weniger als 10 Gew.-%, insbesondere 1 - 9 Gew.-%, besonders bevorzugt weniger als 5 Gew.-%, insbesondere 1 - 4,5 Gew.-%, eine Zellsuspension mit einem Feuchtigkeitsgehalt von 30 bis 60 Gew.-%, insbesondere von 35 bis 50 Gew.-%, eingestellt wird.

Verfahren zur Bestimmung des Feuchtigkeitsgehalts bzw. Feststoffgehalts sind dem Fachmann bekannt. Die Bestimmung des Feststoffgehalts kann beispielsweise unter Verwendung einer Infrarotlampe erfolgen. Vorzugsweise erfolgt die Bestimmung des Feuchtigkeitsgehalts erfindungsgemäß gravimetrisch durch Ermittlung des Wasserverlustes während der thermischen Trocknung, die beispielsweise in einem Trockenschrank durchgeführt werden kann. Feuchtigkeitsgehalt und Feststoffgehalt sind einander komplementär.

Der Zellaufschluss kann unter Einsatz der dem Fachmann bekannten Zellaufschlussverfahren erfolgen, wie beispielsweise durch einen Schneckenextruder, eine Schlägermühle, eine Luftdüsenmühle oder durch Anwendung erhöhten Drucks, beispielsweise durch das sogenannte French-Press-Verfahren.

Der Zellaufschluss wird erfindungsgemäß vorzugsweise unter Verwendung eines Rotor-Stator-Systems oder in einem Extrusionsverfahren durchgeführt.

Das Rotor-Stator-System basiert auf einem stationären Teil, der Stator genannt wird, und einem rotierenden Teil, dem Rotor. Der Rotor besitzt typischerweise eine Umfangsgeschwindigkeit von mindestens 5 m/s, beispielsweise 10 bis 30 m/s, die Spaltbreite zwischen Rotor und Stator kann beispielsweise 0,1 - 0,5 mm betragen. Bei dem Rotor-Stator-System handelt es sich vorzugsweise um ein homogenisierendes System. Als Rotor-Stator kann beispielsweise die Mischmaschine MHD 2000 (IKA) eingesetzt werden. Zur Durchführung des Zellaufschlusses wird die Zell-Suspension in den Zwischenraum zwischen Stator und Rotor zugegeben. In diesem Spalt erfahren die Zellen eine Scherbeanspruchung und zusätzlich entstehen Turbulenzen. Diese beiden Faktoren bewirken den Zellaufschluss.

Der Energieeintrag auf die Zellen, insbesondere unter Verwendung eines Rotor-Stator-Systems oder während des Extrusionsverfahrens, beträgt erfindungsgemäß 0,1- 50 kWh pro Tonne Suspension, wobei der Energieeintrag hierbei vorzugsweise maximal 40, 35 oder 30 kWh pro Tonne Suspension, besonders bevorzugt maximal 25, 20 oder 15 kWh pro Tonne Suspension. Bevorzugte Bereiche stellen hierbei Energieeinträge von 0,1 - 50 kWh pro Tonne Suspension, insbesondere 0,3 - 45 kWh, besonders bevorzugt 0,5 - 40 kWh, insbesondere 0,8 - 35 kWh, vor allem 1 - 30 kWh, insbesondere 1,5 - 25 kWh, 2 - 20 kWh oder 3 - 15 kWh, jeweils pro Tonne Suspension, dar.

Die "Zellaufschlussrate" des erfindungsgemäßen Verfahrens beträgt vorzugsweise mindestens 50 %, besonders bevorzugt mindestens 60, 70 oder 80 %, vor allem mindestens 85, 90 oder 95 %. Unter der "Zellaufschlussrate" ist die Anzahl der aufgeschlossenen Zellen nach Beendigung des Zellaufschlussverfahrens im Verhältnis zur Gesamtzahl der Zellen zu verstehen. Die Zellaufschlussrate kann beispielsweise visuell unter Verwendung eines Mikroskops bestimmt werden als das Verhältnis der Anzahl aufgeschlossener Zellen in Bezug zur Gesamtzahl der Zellen.

Um die Wertstoffe, insbesondere Lipide, gegen oxidativen Abbau zu stabilisieren, können in der Zellsuspension, die für den Zellaufschluss verwendet wird, zusätzlich Antioxidantien enthalten sein. Bevorzugte Antioxidantien stellen hierbei BHT, BHA, TBHA, Ethoxychin, beta-Carotin, Vitamin E und Vitamin C dar. Das Antioxidans ist, sofern eingesetzt, vorzugsweise in einer Menge von 0,01 bis 2 Gew.-% enthalten.

Um den Zellaufschluss zu erleichtern können gegebenenfalls auch geringe Enzymmengen enthalten sein, die zum Verdau der Zellwand beitragen und dadurch die Freisetzung der Lipide erleichtern. Das Zellwand spaltende Enzym ist, sofern eingesetzt, vorzugsweise in einer Menge von 0,01 bis 0,5 Gew.-% in der Zellsuspension enthalten In einer erfindungsgemäß bevorzugten Ausführungsform wird der Zellaufschluss jedoch in Abwesenheit von Enzymen, insbesondere in Abwesenheit von Enzymen, die zum Verdau der Zellwand beitragen, durchgeführt. Denn das Enzym müsste nach seinem Einsatz wieder inaktiviert werden. Außerdem wäre eine größere Wassermenge als erfindungsgemäß bevorzugt erforderlich, um das Enzym effektiv einzusetzen.

Bei den erfindungsgemäß im Zellaufschlussverfahren einzusetzenden Zellen kann es sich um Zellen handeln, die bereits natürlicherweise Wertstoffe, vorzugsweise Lipide, insbesondere PUFAs, produzieren, es kann sich jedoch auch um Zellen handeln, die durch entsprechende gentechnische Verfahren dazu in die Lage versetzt wurden, Lipide, insbesondere PUFAs, zu produzieren. Die Produktion kann hierbei autotroph, mixotroph oder heterotroph erfolgen.

Bevorzugt werden erfindungsgemäß Zellen eingesetzt, die Lipide, insbesondere PUFAs, heterotroph produzieren. Es handelt sich bei den Zellen erfindungsgemäß vorzugsweise um Algen, Pilze, insbesondere Hefen, oder Protisten, es kommen jedoch etwa auch Zellen von öl-produzierenden Pflanzen in Betracht. Besonders bevorzugt handelt es sich um Zellen mikrobieller Algen oder Pilze.

Als Zellen von öl-produzierenden Pflanzen kommen insbesondere die Samen von Soja, Flachs, Raps, Mais, Baumwolle, Distel und Sonnenblume in Betracht.

Als Zellen von öl-produzierenden Hefen kommen insbesondere Stämme von Yarrowia, Candida, Rhodotorula, Rhodosporidium, Cryptococcus, Trichosporon und Lipomyces in Betracht.

Erfindungsgemäß werden vorzugsweise Zellen des Taxons Labyrinthulomycetes (Labyrinthulea, Netzschleimpilze, Schleimnetze) eingesetzt, insbesondere solche der Familie der Thraustochytriaceae. Zu der Familie der Thraustochytriaceae gehören die Gattungen Althomia, Aplanochytrium, Elnia, Japonochytrium, Schizochytrium, Thraustochytrium und Ulkenia. Besonders bevorzugt werden erfindungsgemäß Zellen der Gattungen Thraustochytrium, Schizochytrium und Ulkenia eingesetzt, vor allem solche der Gattung Thraustochytrium oder Schizochytrium. Ein besonders bevorzugt eingesetzter Stamm stellt der Stamm Schizochytrium limacinum SR21 (IFO 32693) dar.

Die erfindungsgemäß einzusetzenden Zellen werden vorzugsweise in Form von sogenannter "Biomasse" eingesetzt. Bei der Biomasse handelt es sich vorzugsweise um das Produkt eines fermentativen Kultivierungsverfahrens. Die Biomasse kann entsprechend neben den aufzuschließenden Zellen auch noch Bestandteile des Fermentationsmediums enthalten. Bei diesen Bestandteilen kann es sich insbesondere um Salze, Antischaummittel und nicht umgesetzte Kohlenstoffquelle und/oder Stickstoffquelle handeln. Der Zellgehalt in dieser Biomasse beträgt vorzugsweise mindestens 70 Gew.-%, vorzugsweise mindestens 75 Gew.-%. Der Zellgehalt in der Biomasse kann gegebenenfalls vor Durchführung des Zellaufschlussverfahrens durch entsprechende Waschschritte beispielsweise auf mindestens 80 oder mindestens 90 Gew.-% erhöht werden. Die erhaltene Biomasse kann jedoch auch direkt in dem Zellaufschlussverfahren eingesetzt werden.

Die erfindungsgemäß im Zellaufschlussverfahren einzusetzenden Zellen zeichnen sich vorzugsweise dadurch aus, dass sie einen Wertstoff-Gehalt, vorzugsweise Lipid-Gehalt, besonders bevorzugt PUFA-Gehalt, von mindestens 20 Gew.-%, vorzugsweise mindestens 30 Gew.-%, insbesondere mindestens 40 Gew.-%, jeweils bezogen auf die Zelltrockenmasse, aufweisen.

In einer bevorzugten Ausführungsform liegt ein Großteil der Lipide in Form von Triglyceriden vor, wobei vorzugsweise mindestens 50 Gew.-%, insbesondere mindestens 75 Gew.-% und in einer besonders bevorzugten Ausführungsform mindestens 90 Gew.-% der in der Zelle enthaltenen Lipide in Form von Triglyceriden vorliegen.

Weiterhin umfassen die in der Zelle enthaltenen Lipide vorzugsweise polyungesättigte Fettsäuren (PUFAs), wobei vorzugsweise mindestens 10 Gew.-%, insbesondere mindestens 20 Gew.-%, besonders bevorzugt 20 bis 60 Gew.-%, insbesondere 20 bis 40 Gew.-%, der in der Zelle enthaltenen Fettsäuren PUFAs darstellen.

Unter polyungesättigten Fettsäuren (PUFAs) werden erfindungsgemäß Fettsäuren verstanden, die mindestens zwei C-C-Doppelbindungen aufweisen. Unter den PUFAs sind erfindungsgemäß hochungesättigte Fettsäuren (HUFAs) bevorzugt. Unter HUFAs werden erfindungsgemäß Fettsäuren verstanden, die mindestens vier C-C-Doppelbindungen aufweisen.

Die PUFAs können in der Zelle in freier Form oder in gebundener Form vorliegen. Beispiele für das Vorliegen in gebundener Form sind Phospholipide und Ester der PUFAs, insbesondere Monoacyl-, Diacyl- und Triacylglyceride. In einer bevorzugten Ausführungsform liegt ein Großteil der PUFAs in Form von Triglyceriden vor, wobei vorzugsweise mindestens 50 Gew.-%, insbesondere mindestens 75 Gew.-% und in einer besonders bevorzugten Ausführungsform mindestens 90 Gew.-% der in der Zelle enthaltenen PUFAs in Form von Triglyceriden vorliegen.

Bevorzugte PUFAs stellen omega-3-Fettsäuren und omega-6-Fettsäuren dar, wobei omega-3-Fettsäuren besonders bevorzugt sind. Bevorzugte omega-3-Fettsäuren stellen hierbei die Eicosapentaensäure (EPA, 20:5ω-3), insbesondere die (5Z,8Z,11Z,14Z,17Z)-Eicosa-5,8,11,14,17-pentaensäure, und die Docosahexaensäure (DHA, 22:6ω-3), insbesondere die (4Z,7Z,10Z,13Z,16Z,19Z)-Docosa-4,7,10,13,16,19-hexaensäure, dar, wobei die Docosahexaensäure besonders bevorzugt ist.

Verfahren zur Herstellung der Lipide, insbesondere PUFAs, enthaltenden Zellen insbesondere der Ordnung Thraustochytriales sind im Stand der Technik ausführlich beschrieben (siehe z.B. WO91/07498, WO94/08467, WO97/37032, WO97/36996, WO01/54510). Die Herstellung erfolgt in der Regel dadurch, dass Zellen in Anwesenheit einer Kohlenstoffquelle und einer Stickstoffquelle in einem Fermenter kultiviert werden. Es können hierbei Biomasse-Dichten von mehr als 100 Gramm pro Liter und Produktionsraten von mehr als 0,5 Gramm Lipid pro Liter pro Stunde erreicht werden. Das Verfahren wird vorzugsweise als sogenanntes Fed-Batch-Verfahren durchgeführt, d.h. dass die Kohlenstoff- und Stickstoffquellen inkrementell während der Fermentation zugeführt werden. Die Lipid-Produktion kann nach Erreichen der gewünschten Biomasse durch unterschiedliche Maßnahmen induziert werden, beispielsweise durch Limitierung der Stickstoffquelle, der Kohlenstoffquelle oder des Sauerstoffgehalts oder Kombinationen davon.

Die Fermentation der Zellen erfolgt vorzugsweise in einem Medium mit niedriger Salinität, insbesondere um Korrosion zu verhindern. Dies kann dadurch erreicht werden, dass anstelle von Natriumchlorid chlor-freie Natriumsalze, wie beispielsweise Natriumsulfat, Natriumcarbonat, Natriumhydrogencarbonat oder Sodaasche, als Natriumquelle verwendet werden. Vorzugsweise wird Chlorid in Mengen von weniger als 3 g/l, insbesondere weniger als 500 mg/l, besonders bevorzugt weniger als 100 mg/l, bei der Fermentation eingesetzt.

Als Kohlenstoffquelle kommen sowohl alkoholische als auch nicht-alkoholische Kohlenstoffquellen in Betracht. Beispiele für alkoholische Kohlenstoffquellen sind Methanol, Ethanol und Isopropanol. Beispiele für nicht-alkoholische Kohlenstoffquellen sind Fructose, Glucose, Saccharose, Molassen, Stärke und Maissirup.

Als Stickstoffquelle kommen sowohl anorganische als auch organische Stickstoffquellen in Betracht. Beispiele für anorganische Stickstoffquellen sind Nitrate und Ammoniumsalze, insbesondere Ammoniumsulfat und Ammoniumhydroxid. Beispiele für organische Stickstoffquellen sind Aminosäuren, insbesondere Glutamat, und Harnstoff.

Zusätzlich können auch anorganische oder organische Phosphorverbindungen und/oder bekannte wachstumsstimulierende Stoffe, wie etwa Hefeextrakt oder Maisquellwasser, hinzugegeben werden, um die Fermentation positiv zu beeinflussen

Die Fermentation der Zellen erfolgt vorzugsweise bei einem pH-Wert von 4 bis 11, insbesondere 6 bis 10, sowie vorzugsweise bei einer Temperatur von mindestens 20°C, insbesondere 20 bis 40°C, besonders bevorzugt mindestens 30 °C. Ein typisches Fermentationsverfahren dauert bis zu etwa 100 Stunden.

Nach Beendigung der Fermentation erfolgt die Ernte der Zellen. Durch Zentrifugation, Filtration, insbesondere Ultra- oder Mikrofiltration, Dekantieren und/oder Lösungsmittelverdampfung kann ein Großteil des Fermentationsmediums von der Biomasse abgetrennt werden. Die Lösungsmittelverdampfung erfolgt vorzugsweise unter Einsatz eines Trommeltrockners, eines Tunneltrockners, durch Sprühtrocknung oder Vakuumverdampfung. Die Lösungsmittelverdampfung kann insbesondere auch unter Einsatz eines Rotationsverdampfers, eines Dünnschichtverdampfers oder eines Fallfilmverdampfers erfolgen. Alternativ zur Lösungsmittelverdampfung kommt beispielsweise auch die Umkehrosmose zur Einengung der Fermentationsbrühe in Betracht. Anschließend wird die erhaltene Biomasse gegebenenfalls weiter getrocknet, vorzugsweise durch Fließbettgranulation. Vorzugsweise wird durch das Trocknen der Feuchtigkeitsgehalt auf unter 15 Gew.-%, insbesondere auf unter 10 Gew.-%, besonders bevorzugt auf unter 5 Gew.-% reduziert.

Vorzugsweise erfolgt nach der Ernte der Zellen oder gegebenenfalls auch schon kurz vor der Ernte der Zellen eine Pasteurisierung der Zellen, um die Zellen abzutöten und Enzyme, die den Abbau der Lipide befördern könnten, zu inaktivieren.

Die auf diese Weise erhaltene - vorzugsweise getrocknete - Biomasse kann nun eingesetzt werden, um die Zellsuspension herzustellen. Zur Herstellung der Suspension wird Wasser oder eine wässrige Lösung eingesetzt.

Die Suspension kann im Falle des Rotor-Stator-Systems entweder direkt im Rotor-Stator-System hergestellt werden, so dass Herstellung der Suspension und Zellaufschluss in einem Schritt erfolgen oder es kann alternativ zunächst in einem Rührsystem die Zellsuspension hergestellt und diese anschließend zwecks Zellaufschluss in dem Rotor-Stator-System eingesetzt werden.

In einer bevorzugten Ausführungsform erfolgt die Herstellung der Suspension im Rotor-Stator-System unter Verwendung eines feststoffmischenden Aufsatzes. Unter einem feststoffmischenden Aufsatz ist hierbei eine Vorrichtung zu verstehen, die das separate Einbringen von einerseits Feststoff und andererseits Wasser bzw. wässriger Lösung in das Rotor-Stator-System erlaubt. Die Suspension wird somit erst während des Zellaufschlusses bzw. unmittelbar vor dem Zellaufschluss durch Vermischung in dem feststoffmischenden Aufsatz hergestellt. Erfindungsgemäß wurde gefunden, dass unter Verwendung eines solchen feststoffmischenden Aufsatzes Suspensionen mit sehr hohen Feststoffgehalten dem Zellaufschluss unterzogen werden können, was mit Hinblick auf die nachfolgende Verarbeitung besonders vorteilhaft ist. Suspensionen, die unter Verwendung eines feststoffmischenden Aufsatzes in dem Rotor-Stator-System eingesetzt werden, weisen vorzugsweise einen Feststoffgehalt von 40-70 Gew.-%, besonders bevorzugt von 50-65 Gew.-%, auf.

Falls zur Herstellung der Zellsuspension eine wässrige Lösung eingesetzt wird, dann kann diese insbesondere weitere Nahrungsmittelkomponenten - wie etwa Vitamine oder Salze enthalten.

Bei dem Wertstoff handelt es sich erfindungsgemäß vorzugsweise um ein Lipid, insbesondere ein Öl, besonders bevorzugt um ein Öl, das polyungesättigte Fettsäuren enthält, wobei vorzugsweise mindestens 10 Gew.-%, insbesondere mindestens 20 Gew.-%, besonders bevorzugt 20 - 60 Gew.-%, vor allem 20 - 40 Gew.-%, der in der Zelle enthaltenden

Lipide polyungesättigte Fettsäuren darstellen.

Weiterer Gegenstand der vorliegenden Erfindung ist ein Verfahren zur Herstellung eines Nahrungs- oder Futtermittels, bei welchem die Zellen nach Durchführung des Zellaufschlusses mit anderen Nahrungs- oder Futtermittelinhaltsstoffen vermischt werden und anschließend zu dem Nahrungs- oder Futtermittel verarbeitet werden.

Die Verarbeitung des Gemisches aus aufgeschlossenen Zellen und weiteren Nahrungs- oder Futtermittelinhaltsstoffen erfolgt in einer bevorzugten Ausführungsform durch ein Extrusionsverfahren, um verkaufsfertige Portionen des Nahrungs- oder Futtermittels zu erhalten. Alternativ kann beispielsweise auch ein Pelletierverfahren eingesetzt werden.

In einer bevorzugten Ausführungsform werden die nicht aufgeschlossenen Zellen zunächst mit den weiteren Nahrungs- oder Futtermittelinhaltsstoffen vermischt, so dass der Zellaufschluss erst während des Extrusionsverfahrens erfolgt.

Weiterer Gegenstand der vorliegenden Erfindung ist daher auch ein Verfahren zur Herstellung eines Nahrungs- oder Futtermittels, bei welchem die Zellen der Zellsuspension vor Durchführung des Zellaufschlusses mit anderen Nahrungs- oder Futtermittelinhaltsstoffen vermischt werden und das so erhaltene Gemisch in einem Extrusionsverfahren zu einem Nahrungs- oder Futtermittel verarbeitet wird.

Im Extrusionsverfahren wird vorzugsweise ein Schnecken- oder Doppelschneckenextruder eingesetzt. Das Extrusionsverfahren wird vorzugsweise bei einer Temperatur von 80 - 220° C, insbesondere 100 - 190 °C, einem Druck von 10 - 40 Bar, und einer Wellenumlaufgeschwindigkeit von 100 - 1000 rpm, insbesondere 300 - 700 rpm, durchgeführt. Die Verweilzeit des eingebrachten Gemisches beträgt vorzugsweise 5 - 30 Sekunden, insbesondere 10 - 20 Sekunden.

Bei einer erfindungsgemäß bevorzugten Art des Extrusionsverfahrens umfasst das Verfahren einen Kompaktierungs- und einen Kompressionsschritt.

Vor der Durchführung des Extrusionsverfahrens werden die Komponenten vorzugsweise innig miteinander vermischt. Dies geschieht vorzugsweise in einer Trommel, die mit Schaufeln ausgestattet ist. Bei diesem Mischungsschritt erfolgt in einer bevorzugten Ausführungsform eine Wasserdampfinjektion, insbesondere um die Quellung der vorzugsweise enthaltenen Stärke zu bewirken.

Die weiteren Nahrungs- oder Futtermittelinhaltsstoffe werden vor dem Vermischen mit den aufgeschlossenen oder nicht aufgeschlossenen Zellen - soweit erforderlich - vorzugsweise zerkleinert, um sicherzustellen, dass bei dem Mischungsschritt ein homogenes Gemisch erhalten wird. Das Zerkleinern der weiteren Nahrungs- oder Futtermittelinhaltsstoffe kann beispielsweise unter Verwendung einer Hammer-Mühle erfolgen.

Ein erfindungsgemäß bevorzugtes Verfahren zur Herstellung eines Nahrungs- oder Futtermittels umfasst daher die folgenden Schritte:
a) Bereitstellung einer Zellsuspension von Zellen der Ordnung Thraustochytriales, insbesondere der Gattung Thraustochytrium oder Schizochytrium, mit einem Feuchtigkeitsgehalt von 30 bis 60 Gew.-%, insbesondere 35 bis 50 Gew.-%, ausgehend von einer Zellmasse mit einem Feuchtigkeitsgehalt von weniger als 15 Gew.-%, vorzugsweise weniger als 10 Gew.-%, insbesondere 1 - 9 Gew.-%, besonders bevorzugt weniger als 5 Gew.-%, insbesondere 1 - 4,5 Gew.-%;
b) Zellaufschluss unter Anwendung eines Energieeintrags von 0,1 - 50 kWh, insbesondere 0,3 - 45 kWh, besonders bevorzugt 0,5 - 40 kWh, insbesondere 0,8 - 35 kWh, vor allem 1 - 30 kWh, insbesondere 1,5 - 25 kWh, 2 - 20 kWh oder 3 - 15 kWh, jeweils pro Tonne Suspension, vorzugsweise unter Verwendung eines Rotor-Stator-Systems;
c) Vermischung der aufgeschlossenen Zellen und/oder daraus isolierter Wertstoffe mit weiteren Nahrungs- oder Futtermittelinhaltsstoffen;
d) Herstellung des finalen Produkts durch ein Kompaktierungs- oder Extrusionsverfahren.

Ein weiteres erfindungsgemäß bevorzugtes Verfahren zur Herstellung eines Nahrungs- oder Futtermittels umfasst die folgenden Schritte:
a) Bereitstellung einer Zellsuspension von Zellen der Ordnung Thraustochytriales, insbesondere der Gattung Thraustochytrium oder Schizochytrium, mit einem Feuchtigkeitsgehalt von 30 bis 60 Gew.-%, insbesondere 35 bis 50 Gew.-%, ausgehend von einer Zellmasse mit einem Feuchtigkeitsgehalt von weniger als 15 Gew.-%, vorzugsweise weniger als 10 Gew.-%, insbesondere 1 - 9 Gew.-%, besonders bevorzugt weniger als 5 Gew.-%, insbesondere 1 - 4,5 Gew.-%;
b) Vermischung der Zellsuspension mit weiteren Nahrungs- oder Futtermittelinhaltsstoffen, wobei die Zellen vorzugsweise 0,5-20 Gew.-%, insbesondere 1-10 Gew.-%, besonders bevorzugt 2-8 Gew.-% der resultierenden Zusammensetzung ausmachen;
c) Herstellung des finalen Produktes durch ein Extrusionsverfahren, wobei ein Energieeintrag von 0,1 - 50 kWh, insbesondere 0,3 - 45 kWh, besonders bevorzugt 0,5 - 40 kWh, insbesondere 0,8 - 35 kWh, vor allem 1 - 30 kWh, insbesondere 1,5 - 25 kWh, 2 - 20 kWh oder 3 - 15 kWh, jeweils pro Tonne Suspension erfolgt.

Erfindungsgemäße Verfahren zur Herstellung eines Nahrungs- oder Futtermittels zeichnen sich vorzugsweise dadurch aus, dass in keinem Verfahrensschritt ein höherer Energieeintrag auf die Zellen bzw. aufgeschlossenen Zellen erfolgt als beim Zellaufschluss. Vorzugsweise ist der Energieeintrag in allen übrigen Verfahrensschritten, denen die Zellen bzw. aufgeschlossenen Zellen bzw. der enthaltene Wertstoff ausgesetzt sind, deutlich geringer als während des Zellaufschlusses. Der Energieeintrag beträgt hierbei in allen anderen Verfahrensschritten vorzugsweise maximal 80 %, insbesondere maximal 60 %, des Energieeintrags, der beim Zellaufschluss auf die Zellen erfolgt. Auf diese Weise wird sichergestellt, dass der enthaltene Wertstoff möglichst wenig geschädigt wird.

Die aufgeschlossenen Zellen machen vorzugsweise 0,5-20 Gew.-%, insbesondere 1-10 Gew.-%, vorzugsweise 2-8 Gew.-% des Nahrungs- oder Futtermittels bzw. der zur Herstellung des Nahrungs- oder Futtermittels eingesetzten Zusammensetzung aus. Weiterer Gegenstand der vorliegenden Erfindung sind auch Nahrungs- oder Futtermittel, die durch erfindungsgemäße Verfahren erhältlich ist.

Die durch zuvor beschriebene, erfindungsgemäße Verfahren erhältlichen Nahrungs- und Futtermittel zeichnen sich vorzugsweise durch eine sehr homogene Verteilung des Wertstoffs in dem erhältlichen Nahrungs- oder Futtermittel aus.. Der Wertstoff macht hierbei vorzugsweise 0,1 - 15 Gew.-%, besonders bevorzugt 0,2 - 8 Gew.-%, des Nahrungs- oder Futtermittels aus.

Die Homogenität der Verteilung kann hierbei auf folgende Weise bestimmt werden: Es werden n Proben mit einer Masse von jeweils x Gramm genommen. Anschließend wird jeweils die in den einzelnen Proben enthaltene Masse des Wertstoffes (y Gramm) bestimmt und der Mittelwert der Masse des in den n Proben enthaltenen Wertstoffes bestimmt (yₙ Gramm).

Durch erfindungsgemäße Verfahren erhaltene Nahrungs- oder Futtermittel zeichnen sich vorzugsweise dadurch aus, dass die Abweichung der enthaltenen Masse des Wertstoffes in den einzelnen Proben in Bezug auf den ermittelten Mittelwert der Masse des Werststoffes maximal 25 %, vorzugsweise maximal 20 oder 10 %, besonders bevorzugt maximal 5, 3 oder 2 % beträgt.

Dieses Merkmal ist hierbei vorzugsweise für mindestens eine der folgenden Vorgaben, vorzugsweise für alle folgenden Vorgaben, erfüllt: 20 oder 50 Stichproben des Nahrungs- oder Futtermittels mit einer Masse von jeweils 1,00 Gramm, 20 oder 50 Stichproben des Nahrungs- oder Futtermittels mit einer Masse von jeweils 500 Milligramm, 20 oder 50 Stichproben des Nahrungs- oder Futtermittels mit einer Masse von jeweils 100 Milligramm.

Weiterhin und/oder alternativ beträgt die Varianz der Verteilung des Wertstoffs in unterschiedlichen Teilportionen des Nahrungs- oder Futtermittels maximal 10 %, vorzugsweise maximal 5 %, insbesondere maximal 3 %.

Die Varianz wird hierbei vorzugsweise ebenfalls ermittelt für 20 oder 50 Stichproben des Nahrungs- oder Futtermittels mit einer Masse von jeweils 1,00 Gramm, 500 Milligramm oder 100 Milligramm, wobei das Merkmal der Varianz vorzugsweise für mindestens eine der Vorgaben, besonders bevorzugt für alle genannten Vorgaben, erfüllt ist.

Bei dem Nahrungs- oder Futtermittel handelt es sich vorzugsweise um ein Mittel zum Einsatz in der Aquakultur oder um ein Nahrungs- oder Futtermittel zum Einsatz in der Geflügelzucht, Schweinezucht oder Rinderzucht. Bei dem Futtermittel kann es sich auch um ein Futtermittel handeln, das eingesetzt wird, um Kleinlebewesen zu züchten, die in der Aquakultur als Futtermittel eingesetzt werden können. Bei den Kleinlebewesen kann es sich beispielsweise um Nematoden, Crustaceen oder Rotiferen handeln. Das Futtermittel liegt vorzugsweise flockenförmig, kugelförmig oder tablettenförmig vor. Ein durch Extrusion erhältliches Futtermittel hat vorzugsweise einen Feuchtigkeitsgehalt von weniger als 5 Gew.-%, besonders bevorzugt von 0,2 bis 4 Gew.-%.

Die anderen Nahrungs- oder Futtermittelinhaltsstoffe sind vorzugsweise ausgewählt aus proteinhaltigen, kohlenhydrathaltigen, nukleinsäurehaltigen und lipidlöslichen Komponenten sowie gegebenenfalls weiteren fetthaltigen Komponenten und weiterhin aus anderen Zusatzstoffen wie Mineralien, Vitaminen, Pigmente und Aminosäuren. Daneben können neben Nährsubstanzen auch strukturgebende Substanzen enthalten sein, um etwa die Textur oder das Erscheinungsbild des Futtermittels zu verbessern. Weiterhin können beispielsweise auch Bindemittel eingesetzt werden, um die Konsistenz des Futtermittels zu beeinflussen. Eine bevorzugt eingesetzte Komponente, die sowohl eine Nährsubstanz als auch eine strukturgebende Substanz darstellt, ist Stärke.

Als proteinhaltige Komponente, die zusätzlich Fette enthält, können beispielsweise eingesetzt werden Fischmehl, Krillmehl, Muschelmehl, Kalmarmehl oder Schrimpsschalen. Als fetthaltige Komponente kann alternativ auch Fischöl eingesetzt werden. Als fetthaltige Komponente kann auch ein Pflanzenöl eingesetzt werden, insbesondere Öl aus Sojabohnen, Rapssamen, Sonnenblumenkernen und Flachssamen. Als kohlenhydrathaltige Komponente kann beispielsweise Weizenmehl, Sonnenblumenmehl, Sojablumenmehl oder Getreidegluten eingesetzt werden.

Der Gesamtgehalt an Öl in dem Futtermittel - inklusive des Öls aus den öl-haltigen Zellen - beträgt vorzugsweise 15 bis 40 Gew.-%, insbesondere 15 bis 30 Gew.-%.

Das Futtermittel zum Einsatz in der Aquakultur wird vorzugsweise verwendet um Flossenfische und Crustaceen zu züchten, die vorzugsweise der Ernährung von Menschen dienen. Hierzu zählen insbesondere Karpfen, Tilapia, Welse, Thunfisch, Lachs, Forellen, Baramundi, Brassen, Barsche, Kabeljau, Schrimps, Hummer, Krabben, Garnelen und Krebse. Besonders bevorzugt handelt es sich um ein Futtermittel für die Lachs-Zucht. Bevorzugte Lachsarten stellen hierbei der Atlantische Lachs, der Rotlachs, der Masu-Lachs, der Königslachs, der Ketalachs, der Silberlachs, der Donaulachs, der Pazifische Lachs und der Buckellachs dar.

Alternativ kann es sich auch um ein Futtermittel handeln, das zur Anzucht von Fischen dient, die anschließend zu Fischmehl oder Fischöl verarbeitet werden. Bei den Fischen handelt es sich hierbei vorzugsweise um Hering, Pollack, Menhaden, Anchovis, Kapelan oder Kabeljau.Das so erhaltene Fischmehl oder Fischöl kann wiederum in der Aquakultur zur Zucht von Speisefischen bzw. Crustaceen eingesetzt werden.

Die Aquakultur kann in Teichen, Tanks, Becken oder auch in abgegrenzten Arealen im Meer oder in Seen, hierbei insbesondere in Käfigen oder Netzpferchen, erfolgen. Die Aquakultur kann dazu dienen, den fertigen Speisefisch heranzuzüchten, jedoch auch eingesetzt werden, um Jungfische heranzuzüchten, die anschließend freigesetzt werden, um den Wildfischbestand aufzustocken.

Bei der Lachszucht erfolgt vorzugsweise zunächst die Heranzucht bis zum Junglachs in Süßwasser-Tanks oder künstlichen Wasserströmen und anschließend die weitere Zucht im Meer in schwimmenden Käfigen bzw. Netzpferchen, die vorzugsweise in Buchten oder Fjorden verankert sind.

### Ausführungsbeispiele

### Beispiel 1: Herstellung einer DHA-enthaltenden Biomasse

Zur Produktion von DHA wurde der Stamm Schizochytrium limacinum SR21 eingesetzt. Dieser ist hinterlegt beim NIBH unter FERM BP-5034 sowie beim IFO unter IFO 32693. Der Stamm S. limacinum SR21 wurde ursprünglich aus Meerwasser isoliert (Nakahara et al. 1996, JAOCS, 73(10); Honda Mycol. Res. 1998).

Die Fermentation des Stammes erfolgte in einem Medium, das 50 % künstliches Meerwasser (Sigma Aldrich) und des Weiteren folgende Komponenten enthielt: 60 g/l Glucose, 0,7 g/l Maisquellwasser (Sigma Aldrich), 2 g/l (NH₄)₂SO₄ und 3 g/l KH₂PO₄.

Die Fermentation erfolgte bei 28°C, einem pH-Wert von 4,0, einer Belüftungsrate von 0,5 vvm und einer Rührung von 200 rpm für 60 Stunden. Nach Beendigung der Fermentation wurde der Fermentationsbrühe ein Antioxidans zugegeben und die Fermentationsbrühe danach mindestens 20 Minuten auf 60°C erhitzt.

Anschließend erfolgte eine zweistufige Trocknung der Biomasse: Zunächst wurde die Fermentationsbrühe durch Verdampfung auf eine Trockenmasse von etwa 20 Gew.-% eingeengt. Anschließend erfolgte Sprühtrocknung der eingeengten Fermentationsbrühe unter Verwendung eines Production Minor™ Spray Dryer (GEA NIRO) bei einer Einlasstemperatur der Trocknungsluft von 340°C. Durch Sprühtrocknung wurde so ein Puder mit einer Trockenmasse von mehr als 95 Gew.-% erhalten.

### Beispiel 2: Aufschluss der getrockneten Biomasse

Der Zellaufschluss der getrockneten Biomasse erfolgte unter Verwendung eines Rotor-Stator-Systems vom Typ MHD 2000 (IKA, Deutschland). Aufgrund eines feststoffmischenden Aufsatzes erlaubt dieses Rotor-Stator-System, die Zellsuspension erst unmittelbar vor dem Zellaufschluss bereitzustellen. D.h. die Vermischung aus Zelltrockenmasse und Wasser erfolgt kurz vor bzw. während der Homogenisierung in situ.

Hierzu wurde das Zellpulver aus Beispiel 1 von oben in den feststoffmischenden Aufsatz hineingegeben und Wasser seitlich zwecks Aufquellung der Zellen hinzudosiert, um hierdurch eine Suspension mit einem Trockenmassegehalt von 55 Gew.-% bzw. 60 Gew.-% bereitzustellen.

Die so in situ hergestellte Zellsuspension wird sofort durch den Rotor-Stator homogenisiert. Mit der homogenisierten Zellsuspension wurde anschließend eine Hexan-Extraktion durchgeführt, um den Gehalt an frei verfügbarer und somit extrazellulärer polyungesättigter Fettsäure DHA zu ermitteln. Zum Vergleich wurde eine Hexan-Extraktion mit der nicht aufgeschlossenen Biomasse durchgeführt. Weiterhin wurde der durchgeführte Zellaufschluss auch optisch mittels Rasterelektronenmikroskop analysiert.

Als Referenz wurde der Gesamtgehalt an DHA in den Zellen durch einen Flammenionisationsdetektor (FID) ermittelt. Hierzu wurden die Zellen einer Probe aufgeschlossen, mit Kaliumhydroxid verseift und anschließend mittels Salzsäure sauer gestellt. Anschließend wurden die freien Fettsäuren mittels BF₃ (Bortrifluorid 30% in Methanol) methyliert und über Verteilungschromatographie mit einem Temperaturgradienten getrennt.

Anschließend erfolgte zur Bestimmung des Gesamtgehalts an DHA die Detektion mit dem Flammenionisationsdetektor.

**Tabelle 1: Parameter für den Zellaufschluss im Rotor-Stator**

| Versuchsreihe | 1 | 2 | 3 | 4 |
|---|---|---|---|---|
| Beabsichtigte Feststoffkonzentration am Ausgang [Gew.-%] | 50 | 50 | 60 | 60 |
| Ermittelte Feststoffkonzentration am Ausgang [Gew.-%] | 49,7 | 51,2 | 59,3 | 59,6 |
| Eingestellter Massenstrom an Wasser [kg/h] | 25 | 25 | 20 | 20 |
| Eingestellter Massenstrom an Biomasse [kg/h] | 25 | 25 | 30 | 30 |
| Umdrehungszahl Rotor-Stator [rpm] | 9985 | 9985 | 9985 | 9985 |
| Temperatur Eingang [°C] | 20 | 20 | 20 | 20 |
| Temperatur Ausgang [°C] | 72 | 73 | 78 | 71 |
| Überdruck Ausgang [bar] | 1,4 | 1,4 | 1,5 | 1,3 |
| Energieeintrag in kWh pro kg Feststoff | 0,05 | 0,05 | 0,06 | 0,06 |
| Energieeintrag in kWh pro Tonne Suspension | 50 | 50 | 36 | 36 |

Zur Durchführung der Hexan-Extraktion wurden jeweils 250 mg Probe in 10 ml-Pyrexröhrchen (Pyrex) eingewogen und mit 1ml internem Standard - in Hexan angesetzt - versetzt. Danach wurde 5 ml Hexan hinzugegeben und die Probe über Nacht geschüttelt. Anschließend wurden 1 ml der so erhaltenen Probe in ein 10 ml-Pyrexröhrchen pipettiert und bei 50°C unter Stickstoff-Begasung im Thermoblock verdampft. Zu dem so erhaltenen Trocknungsrückstand wurden 2 ml 0,5 N KOH zugegeben, das Röhrchen fest verschlossen und die erhaltene Mischung für 15 min bei 100°C im Thermoblock inkubiert und zwischendurch auf einem Vortexmischer gemischt, bis die Glaskugeln sich mindestens eine Umdrehung frei im 10 ml-Pyrexglas drehten. Danach wurde die Probe im Wasserbad auf Raumtemperatur abgekühlt. Anschließend wurde 2 ml 0,7 N HCl und 1 ml BF₃ zugegeben, das Röhrchen wieder fest verschlossen und 15 min bei 100°C im Thermoblock inkubiert und zwischendurch auf dem Vortexmischer gemischt, bis die Glaskugeln sich wieder mindestens eine Umdrehung frei im 10 ml-Pyrexglas drehten. Danach wurde die Probe wieder im Wasserbad auf Raumtemperatur abgekühlt. Anschließend wurden 3 ml Wasser und danach 2 ml Hexan zugeben, die Röhrchen wieder fest verschlossen und durch Schütteln durchmischt. Abschließend wurden die Proben für 1 min bei 4000 rpm zentrifugiert und jeweils 1 ml der oberen Phase in GC-Vials zwecks Analyse überführt. Zum Kalibrieren des GC wurde der Standard GLC 617 der Firma NU-CHEK verwendet

**Tabelle 2: Ergebnisse der Hexanextraktion**

| Versuchsreihe | Freies DHA vor dem Zellaufschluss* | Freies DHA nach dem Zellaufschluss* |
|---|---|---|
| 1 | 25,3 % | 97,8 % |
| 2 | 25,3 % | 99,0 % |
| 3 | 25,3 % | 99,2 % |
| 4 | 25,3 % | 101,6 % |

| | | |
|---|---|---|
| *in Bezug zur FID-Referenz | | |

Die Ergebnisse zur Hexanextraktion belegen, dass sich die Zellen nach dem Aufquellen mit Wasser problemlos durch den Rotor-Stator bei niedrigem Energieeintrag vollständig aufschließen ließen. Dies wurde auch durch die rasterelektronenmikroskopische Aufnahme bestätigt, auf welcher keine intakten Zellen mehr zu erkennen waren.

Das Aufquellen der Zellen stellt somit ein effektives Mittel dar, um den Aufschluss der Zellen bei niedrigem Energieeintrag zu ermöglichen. Durch den relativ niedrigen Wassereintrag vor dem Zellaufschluss wird zugleich sichergestellt, dass das erhaltene Produkt ohne weitere Aufkonzentrierung zu einem Futtermittel weiterverarbeitet werden kann

## Patentansprüche

1. Verfahren zum Aufschluss von mikrobiellen Zellen, die einen Wertstoff enthalten, **dadurch gekennzeichnet, dass** die Zellen vor dem Zellaufschluss aufgequollen werden, wobei bei Durchführung des Zellaufschlusses ein Energieeintrag auf die Zellsuspension von 0,1 bis 50 kWh/t erfolgt, **dadurch gekennzeichnet, dass** das Aufquellen der Zellen dadurch erfolgt, dass ausgehend von einer Zellmasse mit einem Feuchtigkeitsgehalt von weniger als 15 Gew.-% eine Zellsuspension mit einem Feuchtigkeitsgehalt von 30 bis 60 Gew.-% eingestellt wird.

2. Verfahren nach Anspruch 1, **dadurch gekennzeichnet, dass** es sich bei dem Wertstoff um ein Lipid handelt, wobei das Lipid vorzugsweise mindestens 20 Gew.-% der Zellmasse ausmacht und vorzugsweise polyungesättigte Fettsäuren enthält.

3. Verfahren nach Anspruch 1 oder 2, **dadurch gekennzeichnet, dass** das Aufquellen der Zellen dadurch erfolgt, dass ausgehend von einer Zellmasse mit einem Feuchtigkeitsgehalt von weniger als 15 Gew.-%, vorzugsweise weniger als 10 Gew.-%, insbesondere weniger als 5 Gew.-%, eine Zellsuspension mit einem Feuchtigkeitsgehalt von 30 bis 60 Gew.-%, vor allem 35 bis 50 Gew.-%, eingestellt wird.

4. Verfahren nach einem der vorhergehenden Ansprüche, **dadurch gekennzeichnet, dass** zur Durchführung des Zellaufschlusses ein Rotor-Stator-System eingesetzt wird.

5. Verfahren nach einem der Ansprüche 1 bis 3, **dadurch gekennzeichnet, dass** der Zellaufschluss in einem Extrusionsverfahren erfolgt.

6. Verfahren nach Anspruch 5, **dadurch gekennzeichnet, dass** die Zellen vor Durchführung des Extrusionsverfahrens mit anderen Nahrungs- oder Futtermittelinhaltsstoffen vermischt werden und im Extrusionsverfahren zu einem Nahrungs- oder Futtermittel verarbeitet werden.

7. Verfahren nach einem der vorhergehenden Ansprüche, **dadurch gekennzeichnet, dass** bei Durchführung des Zellaufschlusses ein Energieeintrag auf die Zellsuspension von 0,5 - 40 kWh/t, insbesondere 1 - 30 kWh/t, vor allem 3 - 15 kWh/t, erfolgt.

8. Verfahren nach einem der vorhergehenden Ansprüche, **dadurch gekennzeichnet, dass** es sich bei den mikrobielle Zellen um Labyrinthulomyceten, vorzugsweise der Familie der Thraustochytriaceae, besonders bevorzugt der Gattungen Thraustochytrium, Schizochytrium oder Ulkenia, handelt.

9. Verfahren nach einem der vorherigen Ansprüche, **dadurch gekennzeichnet, dass** die Zellen 0,5-20 Gew.-%, insbesondere 1-10 Gew.-%, vorzugsweise 2-8 Gew.-%, der im Extrusionsverfahren eingesetzten Zusammensetzung ausmachen und die anderen Nahrungs- oder Futtermittelinhaltsstoffe ausgewählt sind aus kohlenhydrathaltigen, proteinhaltigen, nukleinsäurehaltigen, lipidlöslichen und gegebenenfalls weiteren fetthaltigen Komponenten

10. Verfahren zur Herstellung eines Nahrungs- oder Futtermittels, folgende Schritte umfassend
a) Bereitstellung einer Zellsuspension von Zellen der Ordnung Thraustochytriales, insbesondere der Gattung Thraustochytrium oder Schizochytrium, mit einem Feuchtigkeitsgehalt von 30 bis 60 Gew.-%, insbesondere 35 bis 50 Gew.-%, ausgehend von einer Zellmasse mit einem Feuchtigkeitsgehalt von weniger als 15 Gew.-%, vorzugsweise weniger als 10 Gew.-%, insbesondere 1 - 9 Gew.-%, besonders bevorzugt weniger als 5 Gew.-%, insbesondere 1 - 4,5 Gew.-%,
b) Zellaufschluss unter Anwendung eines Energieeintrags von 0,1 - 50 kWh, insbesondere 0,3 - 45 kWh, besonders bevorzugt 0,5 - 40 kWh, insbesondere 0,8 - 35 kWh, vor allem 1 - 30 kWh, insbesondere 1,5 - 25 kWh, 2 - 20 kWh oder 3 - 15 kWh, jeweils pro Tonne Suspension, vorzugsweise unter Verwendung eines Rotor-Stator-Systems;
c) Vermischung der aufgeschlossenen Zellen und/oder daraus isolierter Wertstoffe mit weiteren Nahrungs- oder Futtermittelinhaltsstoffen;
d) Herstellung des finalen Produkts durch ein Kompaktierungs- oder Extrusionsverfahren.

## Claims

1. Process for disrupting microbial cells containing a substance of value, **characterized in that** the cells are swollen before the cell disruption, an energy input on the cell suspension of 0.1 to 50 kWh/t being effected when carrying out the cell disruption, **characterized in that** the swelling of the cells is effected by setting a cell suspension having a moisture content of 30% to 60% by weight starting from a cell mass having a moisture content of less than 15% by weight.

2. Process according to Claim 1, **characterized in that** the substance of value is a lipid, the lipid preferably accounting for at least 20% by weight of the cell mass and preferably containing polyunsaturated fatty acids.

3. Process according to Claim 1 or 2, **characterized in that** the swelling of the cells is effected by setting a cell suspension having a moisture content of 30% to 60% by weight, especially 35% to 50% by weight, starting from a cell mass having a moisture content of less than 15% by weight, preferably less than 10% by weight and in particular less than 5% by weight.

4. Process according to any of the preceding claims, **characterized in that** a rotor-stator system is used for carrying out the cell disruption.

5. Process according to any of Claims 1 to 3, **characterized in that** the cell disruption is effected in an extrusion process.

6. Process according to Claim 5, **characterized in that** the cells are mixed with other foodstuff or feedstuff ingredients before carrying out the extrusion process and are processed in the extrusion process to form a foodstuff or feedstuff.

7. Process according to any of the preceding claims, **characterized in that** an energy input on the cell suspension of 0.5-40 kWh/t, in particular 1-30 kWh/t and especially 3-15 kWh/t is effected when carrying out the cell disruption.

8. Process according to any of the preceding claims, **characterized in that** the microbial cells are Labyrinthulomycetes, preferably from the family of the Thraustochytriaceae, particularly preferably from the genera Thraustochytrium, Schizochytrium or Ulkenia.

9. Process according to any of the preceding claims, **characterized in that** the cells account for 0.5% - 20% by weight, in particular 1% - 10% by weight and preferably 2% - 8% by weight of the composition used in the extrusion process and the other foodstuff or feedstuff ingredients are selected from carbohydrate-containing, protein-containing, nucleic acid-containing, lipid-soluble and optionally further fat-containing components.

10. Process for producing a foodstuff or feedstuff, comprising the following steps
a) provision of a cell suspension of cells from the order Thraustochytriales, in particular from the genus Thraustochytrium or Schizochytrium, having a moisture content of 30% to 60% by weight, in particular 35% to 50% by weight, starting from a cell mass having a moisture content of less than 15% by weight, preferably less than 10% by weight, in particular 1% - 9% by weight, and particularly preferably less than 5% by weight, in particular 1% - 4.5% by weight;
b) cell disruption with application of an energy input of 0.1 - 50 kWh, in particular 0.3 - 45 kWh, particularly preferably 0.5 - 40 kWh, in particular 0.8 - 35 kWh, especially 1 - 30 kWh and in particular 1.5 - 25 kWh, 2 - 20 kWh or 3 - 15 kWh, per tonne of suspension in each case, preferably with use of a rotor-stator system;
c) mixing of the disrupted cells and/or substances of value isolated therefrom with further foodstuff or feedstuff ingredients;
d) production of the final product by a compaction or extrusion process.

## Revendications

1. Procédé de désintégration de cellules microbiennes, qui contiennent une substance de valeur, **caractérisé en ce que** les cellules sont gonflées avant la désintégration cellulaire, un apport d'énergie à la suspension cellulaire de 0,1 à 50 kWh/t ayant lieu lors de la réalisation de la désintégration cellulaire, **caractérisé en ce que** le gonflement des cellules a lieu en ce qu'à partir d'une masse cellulaire ayant une teneur en humidité de moins 15 % en poids, une suspension cellulaire ayant une teneur en humidité de 30 à 60 % en poids est ajustée.

2. Procédé selon la revendication 1, **caractérisé en ce que** la substance de valeur consiste en un lipide, le lipide représentant de préférence au moins 20 % en poids de la masse cellulaire et contenant de préférence des acides gras polyinsaturés.

3. Procédé selon la revendication 1 ou 2, **caractérisé en ce que** le gonflement des cellules a lieu **en ce qu'**à partir d'une masse cellulaire ayant une teneur en humidité de moins de 15 % en poids, de préférence de moins de 10 % en poids, notamment de moins de 5 % en poids, une suspension cellulaire ayant une teneur en humidité de 30 à 60 % en poids, avant tout de 35 à 50 % en poids, est ajustée.

4. Procédé selon l'une quelconque des revendications précédentes, **caractérisé en ce qu'**un système rotor-stator est utilisé pour la réalisation de la désintégration cellulaire.

5. Procédé selon l'une quelconque des revendications 1 à 3, **caractérisé en ce que** la désintégration cellulaire a lieu dans un procédé d'extrusion.

6. Procédé selon la revendication 5, **caractérisé en ce que** les cellules sont mélangées avant la réalisation du procédé d'extrusion avec d'autres constituants d'aliments ou d'aliments pour animaux, et transformées en un aliment ou aliment pour animaux dans le procédé d'extrusion.

7. Procédé selon l'une quelconque des revendications précédentes, **caractérisé en ce qu'**un apport d'énergie à la suspension cellulaire de 0,5 à 40 kWh/t, notamment de 1 à 30 kWh/t, avant tout de 3 à 15 kWh/t, a lieu lors de la réalisation de la désintégration cellulaire.

8. Procédé selon l'une quelconque des revendications précédentes, **caractérisé en ce que** les cellules microbiennes consistent en des labyrinthulomycètes, de préférence de la famille des Thraustochytriaceae, de manière particulièrement préférée des genres Thraustochytrium, Schizochytrium ou Ulkenia.

9. Procédé selon l'une quelconque des revendications précédentes, **caractérisé en ce que** les cellules représentent 0,5 à 20 % en poids, notamment 1 à 10 % en poids, de préférence 2 à 8 % en poids, de la composition utilisée dans le procédé d'extrusion, et les autres constituants d'aliments ou d'aliments pour animaux sont choisis parmi les composants contenant des glucides, contenant des protéines, contenant des acides nucléiques, solubles dans les lipides et éventuellement d'autres composants contenant des matières grasses.

10. Procédé de fabrication d'un aliment ou aliment pour animaux, comprenant les étapes suivantes :
a) la préparation d'une suspension cellulaire de cellules de l'ordre des Thraustochytriales, notamment du genre Thraustochytrium ou Schizochytrium, ayant une teneur en humidité de 30 à 60 % en poids, notamment de 35 à 50 % en poids, à partir d'une masse cellulaire ayant une teneur en humidité de moins de 15 % en poids, de préférence de moins de 10 % en poids, notamment de 1 à 9 % en poids, de manière particulièrement préférée de moins de 5 % en poids, notamment de 1 à 4,5 % en poids,
b) la désintégration cellulaire en utilisant un apport d'énergie de 0,1 à 50 kWh, notamment 0,3 à 45 kWh, de manière particulièrement préférée 0,5 à 40 kWh, notamment 0,8 à 35 kWh, avant tout 1 à 30 kWh, notamment 1,5 à 25 kWh, 2 à 20 kWh ou 3 à 15 kWh, à chaque fois par tonne de suspension, de préférence en utilisant un système rotor-stator ;
c) le mélange des cellules désintégrées et/ou de substances de valeur isolées à partir de celles-ci avec d'autres constituants d'aliments ou d'aliments pour animaux ;
d) la fabrication du produit final par un procédé de compactage ou d'extrusion.
